# EUROPEAN PATENT SPECIFICATION

(11) **EP 2 147 650 B1**
(45) Date of publication and mention of the grant of the patent: **09.10.2013**
(21) Application number: 09009493.9
(22) Date of filing: 22.07.2009
(51) Int. Cl.: A61B 18/14

(54) **High-frequency treatment instrument**
Hochfrequenzbehandlungsinstrument
Instrument de traitement des hautes fréquences

(30) Priority: 23.07.2008 JP 2008189686
(43) Date of publication of application: 27.01.2010
(73) Proprietor: Olympus Medical Systems Corp., Tokyo 151-0072 (JP)
(72) Inventor: Yamamoto, Tetsuya, Tokyo (JP); Watanabe, Hiroyoshi, Tokyo (JP); Kimura, Megumi, Tokyo (JP)
(74) Representative: von Hellfeld, Axel

(56) References cited:
- EP-A- 1 847 231
- EP-A- 1 867 296
- EP-A- 1 875 854
- EP-A- 1 994 904
- WO-A-94/17741
- WO-A-02/080799
- DE-A1-102005 040 386
- US-A- 5 746 740
- US-A1- 2002 123 667
- US-A1- 2007 149 971

## Description

### BACKGROUND OF THE INVENTION

### Field of the Invention

The present invention relates to a high-frequency treatment instrument that is used with high-frequency electric current applied therethrough.

### Description of the Related Art

High-frequency treatment instruments, for performing various procedures relative to tissues inside of the body cavity of the patient by applying high-frequency electric current from a high-frequency power source to a place to be treated, are conventionally known.

As a typical high-frequency treatment instrument, high-frequency forceps, which is described in Patent Document 1, is known. In the high-frequency forceps, when an operation wire that is connected to a proximal end of a pair of forceps for performing procedure inside of the body is extended and retracted, the pair of forceps can be opened and closed.

In the high-frequency forceps, both the forceps pieces and the operation wire are made of metal which is a conductive material. A proximal end portion of the operation wire is connected to the high-frequency power source at an operation portion, where an operator operates the operation wire, and supplied high-frequency electric current flows to the forceps pieces by passing through the operation wire.
Patent Document 1: Japanese Unexamined Patent Application, JP 2005-058 344.

However, the pair of forceps pieces of the high-frequency forceps disclosed in Patent Document 1 is rotatably connected to a pivoted portion provided in the proximal end portions of the forceps pieces. Therefore, when the forceps pieces open by operating the operation wire, the proximal ends of the forceps pieces, to which the operation wire is connected, project by opening around the pivoted portion. Accordingly, the proximal ends of the forceps pieces tend to touch tissues inside of the body cavity. Since the high-frequency electric current is supplied to the forceps pieces and the operation wire as described above, when the proximal ends of the forceps pieces and nearby operation wire touch the tissues, the high-frequency electric current leaks from the touched portion. This causes issues which decrease efficiency of the procedures of the forceps pieces.

EP 1 847 231 A1 discloses a soft bipolar forceps, into which a pair of operating wires have been inserted and a pair of forceps pieces, which extend in axial direction and are mutually insulated as well as disposed opposite one another, are connected to a distal end of a flexible cube, via are connecting number. A tip cover supports the forceps pieces, to permit relatively free opening and closing thereof. An operator for advancing were retracting the paired operating wires with respect to the flexible cube is also provided. A first through hole is formed in the approximate centre of a first connector to permit passage through the first connector in cross direction. A conductive part is provided to the base end of the first connector and the operating wire is connected thereto. The forceps piece is further provided with a second arm that extends further than first arm and a second electrode is disposed on the second arm. The tip cover consists of an insulated member formed of a ceramic.

EP 1 994 904 A1 discloses a high frequency surgical instrument comprising a forceps assembly composed of a pair of opening and closing grasper members. The entire body of the grasper member, which is made of a conducting material, functions as an electrode. An operating wire is threaded internally to a coil sleeve and connected to a drive member at its four distal end. The proximal end of the operating wire is extended into a manipulation handle from a rear end of a flexible core. The operating wire is a conducting wire and surrounded by an insolating cover or a case.

US 2007/0149971 A1 discloses a bipolar high-frequency treatment device comprising a first forceps blade and a second forceps blade. The first and second forceps blade and are respectively made of a metal such as stainless steel. The entire surfaces other than the contacting face may be covered with an insolating coating layer. Furthermore, the entire parts other than the contacting face of the first forceps blade and of the second forceps blade may be made of an insolating material.

US 5,756,740 disclosed a surgical biopsy forceps apparatus comprising an endoscopic portion, which is electrically conductive and the apparatus is provided with conducting means attachable to a frame and in electrical communication with the endoscopic portion for receiving an electrical current from an electrical source. The jaw members are electrically conductive. The insolating means is applied to the jaw members.

The present invention was achieved in view of the forgoing circumstances and has an objective to prevent the high-frequency electric current from leaking except from portions where the procedures are performed and to provide a high-frequency treatment instrument capable of performing efficiently.

### SUMMARY OF THE INVENTION

A first aspect of the present invention is a high-frequency treatment instrument that is used with high-frequency electric current supplied from a power source, which includes: a pair of forceps members connected with each other by a rotational axis; and an operation wire having a distal end portion which is rotationally connected to proximal end portions of the forceps members and a proximal end portion which is electrically connected with the power source, in which at least one of the pair of forceps members has a conductive electrode portion, and an insulating portion disposed so as to cover at least part of the electrode portion, the operation wire is electrically connected with the electrode portions, and a conductive outer surface of the forceps members, which includes a portion where the operation wire and the electrode portion are electrically connected, located closer to the proximal end portions of the forceps members than the rotational axis of the forceps members is covered by the insulating portion so as not to be exposed.

In accordance with the first aspect of the high-frequency treatment instrument of the present invention, the insulating portion prevents the high-frequency electric current supplied to the forceps members from leaking from the conductive outer surface located closer to the proximal end than the rotational axis.

The operation wire may have an annular connection portion formed at the distal end portion, and the electrode portion may have a locking projection formed closer to the proximal end portion than the rotational axis. The operation wire and the electrode portion may be electrically connected by the locking projection being inserted through the connection portion. In this case, the operation wire is preferably prevented from falling out from the forceps members.

The electrode portion may have a fitting hole formed closer to the proximal end portion than the rotational axis, and the operation wire may have a fitting axis attached to the distal end portion. The operation wire and the electrode portion may be electrically connected by the fitting axis fitting rotationally into the fitting hole. In this case, it is possible to increase assemblability between the operation wire and the electrode portion.

A second aspect of the present invention is a high-frequency treatment instrument that is used with high-frequency electric current supplied from a power source, which includes: a pair of forceps members connected with each other by a rotational axis; and an operation wire having a distal end portion which is rotationally connected to proximal end portions of the pair of forceps members and proximal end a portion which is electrically connected with the power source, in which at least either of the pair of forceps members has a conductive electrode portion for performing procedures and an insulating portion disposed so as to cover at least a part of the electrode portion, the insulating portion covers an outer surface of the forceps member having the electrode portion closer to the proximal end portion of the forceps member than the rotational axis, a penetrating hole penetrating the insulating portion and the electrode portion is formed in the proximal end portion of the forceps member having the electrode portion, and an area including at least the distal end of the operation wire except a portion located in the penetrating hole is insulatingly covered and inserted through the penetrating hole and the operation wire and the electrode portion are electrically connected in the penetrating hole.

In accordance with the second aspect of the high-frequency treatment instrument of the present invention, since the operation wire is insulatingly covered excluding the portion where the operation wire and the electrode portion are electrically conducted, the high-frequency electrical current supplied to the forceps members is prevented from leaking except from the electrode portion.

In accordance with the high-frequency treatment instrument of the present invention, since the high-frequency electrical current that is supplied is prevented from leaking except from a portion for performing procedures, it is possible to effectively perform procedures.

### BRIEF DESCRIPTION OF THE DRAWINGS

FIG 1 shows an overview of a high-frequency treatment instrument in accordance with a first embodiment of the present invention.
FIG 2A shows a second forceps member of the high-frequency treatment instrument in accordance with the first embodiment of the present invention.
FIG 2B shows the second forceps member viewed from the bottom of FIG. 1.
FIG 3 shows a connection portion connecting the second forceps member and the operation wire.
FIG 4 shows a perspective exploded view of the connection portion.
FIG 5 shows an operation of the high-frequency treatment instrument for use.
FIG 6 shows a connection portion connecting between a second forceps member and an operation wire of a high-frequency treatment instrument in accordance with a second embodiment of the present invention.
FIG 7 is a cross sectional view of the connection portion.
FIG 8 is a perspective exploded view showing a connection portion connecting between a second forceps member and an operation wire of an alternative example of the high-frequency treatment instrument in accordance with the second embodiment of the present invention.
FIG 9 is a cross sectional view of the connection portion.
FIG 10 shows a second forceps member and an operation wire of a high-frequency treatment instrument in accordance with a third embodiment of the present invention.
FIG 11 is a perspective exploded view showing a connection portion connecting the second forceps member and the operation wire.
FIG 12 is a cross sectional view of the connection portion.
FIG 13 is a perspective exploded view showing a connection portion connecting between a second forceps member and an operation wire of an alternative example of the high-frequency treatment instrument in accordance with the third embodiment of the present invention.
FIG 14 is a cross sectional view of the connection portion.

### DETAILED DESCRIPTION OF THE INVENTION

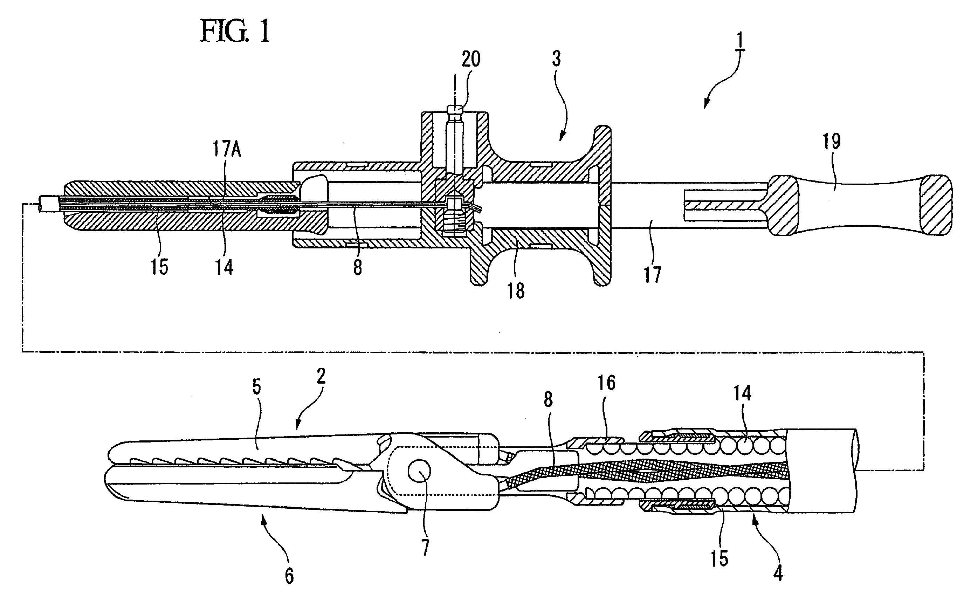

Hereinbelow, a high-frequency treatment instrument in accordance with a first embodiment of the present invention shall be described with reference to FIGS. 1 to 5. The high-frequency treatment instrument 1 in accordance with the present embodiment is used with high-frequency electric current being applied from a power source (not shown). As shown in FIG 1, the high-frequency treatment instrument 1 includes a treatment portion 2 that performs procedures relative to tissues inside of the body cavity, an operation portion 3 that operates the treatment portion 2, and an insertion portion 4 that connects the treatment portion 2 and the operation portion 3.

A first forceps member 5 and a second forceps member 6 of a pair of forceps members are rotatably coupled at a rotation axis 7 in a proximal end portion of the treatment portion 2. Operation wires 8 are connected to each of the forceps members 5 and 6 at positions closer to proximal ends than the rotation axis 7. The operation wires 8 pass through the insertion portion 4 and are connected to the operation portion 3.

The first forceps member 5 located on the upper side of FIG 1 is made of ceramic such as alumina or zirconia or a resin such as PTFE (polytetrafluoroethylene) or PEEK (registered trademark) and has insulation properties. Instead of such composition, the first forceps member 5 may also be made of metal such as stainless steel and may be covered with an insulating coating on the surface.

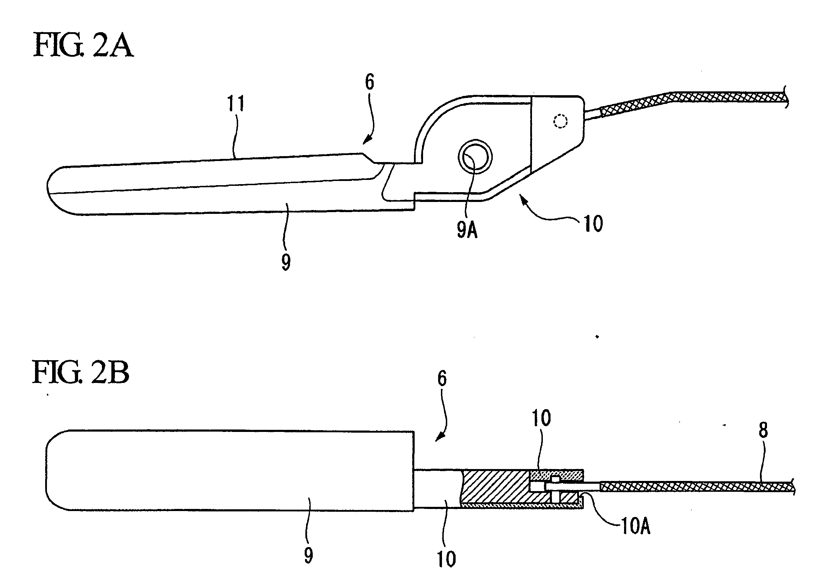

FIG 2A shows the second forceps member 6 located on the lower side of FIG. 1. FIG. 2B shows the second forceps member 6 viewed from the bottom of FIG 1. The second forceps member 6 has an electrode portion 9, which is used for performing procedures, made of a conductive material such as stainless steel and an insulating portion 10 made of the same material as the first forceps member 5.

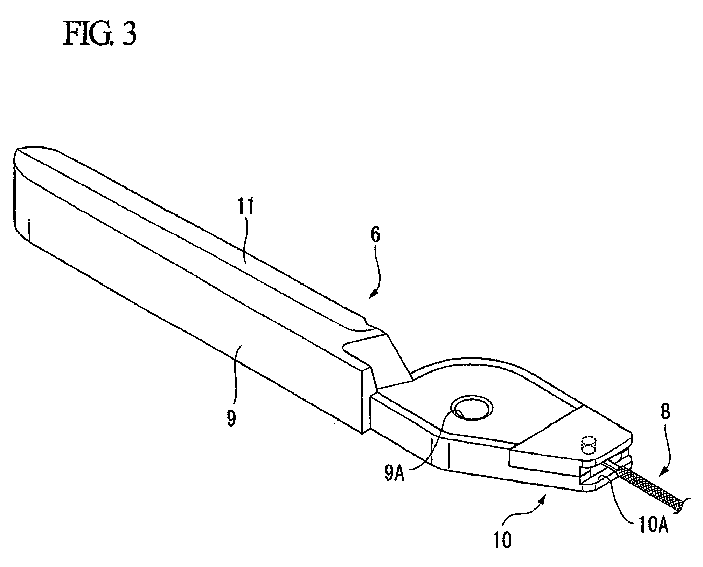

A movable area closer to a distal end portion of the second forceps member 6 than the rotation axis 7 is made of substantially the electrode portion 9 only. A portion of the second forceps member 6 facing the distal end portion of the first forceps member 5 functions as a treatment electrode 11 that performs treatment relative to the tissues inside of the body cavity. On the other hand, an area of the electrode portion 9 closer to the proximal end portion of the electrode portion 9 than the treatment electrode 11 including the periphery of an axial hole 9A in which the rotation axis 7 passes is sandwiched by insulating portions 10 as shown in FIGS. 2B and 3. Accordingly, since the insulating portion 10 covers the area, conductive outer surfaces thereof are not exposed when assembled.

A distal end of one of the operation wires 8 is inserted from a notch 10A disposed in the insulating portion 10 and is electrically connected to the electrode portion 9. The operation wire 8 is insulatingly covered excluding the part thereof which is inserted in the notch 10A. The insulating covering may be made by using insulating tube or insulating coating, for example.

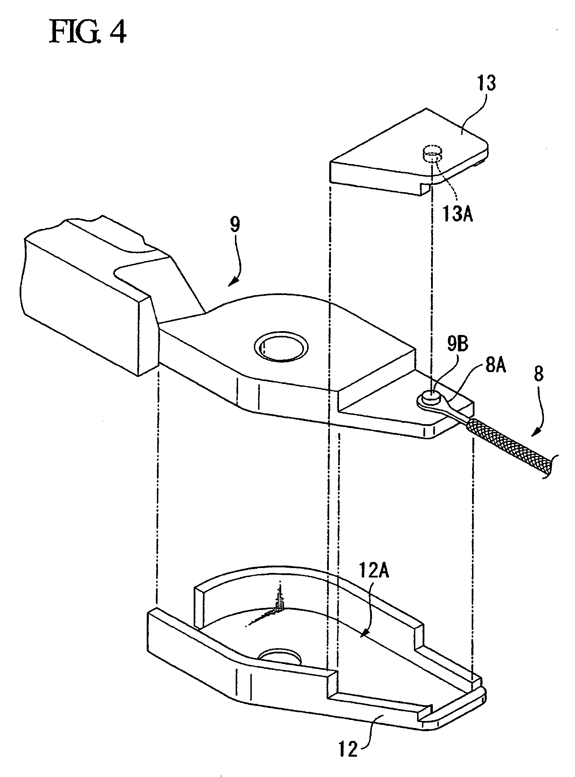

FIG 4 shows a perspective exploded view of the connection portion between the operation wire 8 and the electrode portion 9. The distal end (connection portion) 8A of the operation wire 8 is formed in annular shape. The operation wire 8 is locked by the electrode portion 9 by a projection (locking projection) 9B disposed projectingly in the electrode portion 9 inserting the distal end 8A. The proximal end portion of the electrode portion 9 is sandwiched and covered by a first insulating member 12 and a second insulating member 13 which form the insulating portion 10.

The first insulating member 12 located on the lower side of FIG 4 has a concave portion 12A the figure of which corresponds to the electrode portion 9. On the other side, the second insulating member 13 located in upper side in FIG 4 has a fitting hole 13A that can house the projection 9B.

That is, the proximal end area of the electrode portion 9 formed in a substantially plate shape is housed inside of the concave portion 12A of the first insulating member 12. Furthermore, the proximal area of the electrode portion 9 is sandwiched between the first insulating member 12 and the second insulating member 13 with the projection 9B inserted through the distal end 8A of the operation wire 8 being fitted into the fitting hole 13A of the second insulating member 13.

Accordingly, the area of the second forceps member 6 closer to the proximal end portion thereof than the treatment electrode 11 is insulatingly covered by the insulating portion 10 without increasing the thickness thereof. The operation wire 8 electrically connected to the electrode portion 9 is rotatable relative to the second forceps member 6 within the range of the notch 10A. Excluding the point that the first forceps member 5 does not have the electrode portion 9, the operation wire 8 and the proximal end area of the first forceps member 5 are rotatably connected in substantially the same aspect as the second forceps member 6.

The insertion portion 4 is provided with a coil sheath 14 and an insulating tube 15 covering the outer circumference of the coil sheath 14. A distal end of the coil sheath 14 is connected with the rotation axis 7 via a connection member 16. The rotation axis 7 is not movable relative to the insertion portion 4.

The operation portion 3 is provided with an elongated operation portion main body 17 and a slider 18 fixed so as to be movable in the axial line direction relative to the operation portion main body 17. In the operation portion main body 17, a hole for the insertion portion 17A, in which the insertion portion 4 is inserted, is provided. The coil sheath 14 and the insulating tube 15 are inserted in the hole for the insertion portion 17A and proximal ends thereof are connected. A pair of operation wires 8, which is inserted in the hole for the insertion portion 17A, is connected to the slider 18. A handle 19 is provided in the proximal end of the operation portion main body 17.

A plug 20, to which a power source cable (not shown) that is connected with the high-frequency power source (not shown) is to be connected, is fixed to the slider 18. Accordingly, when the high-frequency power source is connected to the plug 20 via the power source cable and the electricity is turned on, the high-frequency electric current is supplied to the electrode portion 9 by passing the operation wire 8.

Movement of the high-frequency treatment instrument 1 constituted as described above during use shall be described.

An endoscope (not shown) is inserted in a body of a patient, with which a publicly known return electrode (not shown) is in touch, the distal end of the endoscope is advanced to the vicinity of the tissues inside of the body cavity which is a target of the treatment.

Next, the slider 18 is retracted relative to the operation portion main body 17 so that the pair of forceps members 5 and 6 is closed and the insertion portion 4 is inserted to a forceps channel (not shown). After protruding the treatment portion 2 from the forceps channel, the high-frequency power source and the plug 20 are connected with the power source cable.

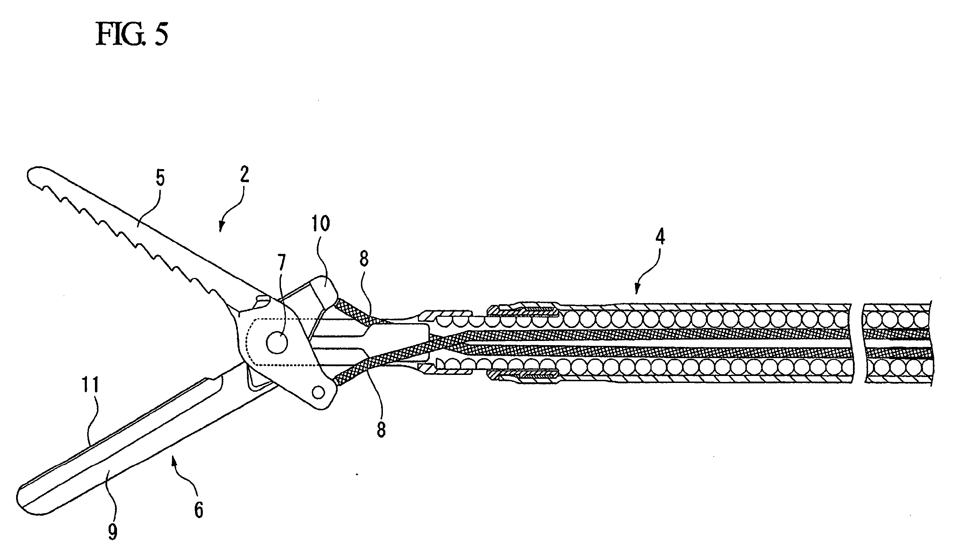

When performing treatments, the slider 18 is advanced relative to the operation portion main body 17. Then the operation wire 8 connected to the slider 18 advances relative to the coil sheath 14. As described above, since the rotation axis 7 is not movable relative to the insertion portion 4, the first forceps member 5 and the second forceps member 6 rotate around the rotation axis 7 and the treatment portion 2 opens as shown in FIG 5.

Along with the opening of the treatment portion 2, areas close to the proximal end portions of the forceps members 5 and 6 which are closer to the proximal ends thereof than the rotation axis 7 project so as to depart from the axial line of the insertion portion 4. Furthermore, areas close to the distal end of the operation wire 8, which is connected to the proximal end portions of the forceps members 5 and 6, departs from the axial line of the insertion portion 4.

However, the outer surface of the area close to the proximal end of the second forceps member 6 is insulatingly covered by the insulating portion 10 and the area close to the distal end of the operation wire 8 is insulatingly covered. Accordingly, even when these portions touch tissues inside of the body cavity, which are not targets of treatments, with the treatment portion 2 being supplied with electricity, the high-frequency electric current does not leak.

When an operator locates the objective tissue between the forceps members 5 and 6 of the treatment portion 2, which is open, and retracts the slider 18 to the proximal end portion of the operation portion main body 17, the distal end portions of the pair of forceps members 5 and 6 including the treatment electrode 11 close again, and the target tissue is sandwiched by the treatment portion 2.

In this state, when the operator supplies the high-frequency electric current from the high-frequency power source, the high-frequency electric current is supplied to the electrode portion 9 by passing the operation wire 8 and the target tissue is cauterized by the high-frequency electric current at the treatment electrode 11.

After the treatment, the operator removes the high-frequency treatment instrument 1 from the forceps channel, removes the endoscope from the body, and finishes the procedures.

In accordance with the high-frequency treatment instrument 1 of the present embodiment, when the treatment portion 2 opens, proximal end areas of the forceps members 5 and 6, which easily touch tissues that are not targets of the treatment, have insulation properties, with the first forceps member 5 being made of insulating material, and the second forceps member 5 being covered with the insulating portion 10. The distal end area of the operation wire 8, which departs from the axial line of the insertion portion 4 when the treatment portion 2 opens, and which easily touch tissues that are not targets of the treatment, are also insulatingly covered.

Accordingly, even when those portions touch tissues, the high-frequency electric current does not leak and the electric current efficiently concentrates on the treatment electrode 11, it is possible to improve the efficiency of the treatment.

The operation wire 8 and the electrode portion 9 are physically and electrically connected with the distal end 8A of the operation wire 8 being formed in an annular shape and fixed to the projection 9B of the electrode portion 9, and the projection 9B fitting the fitting hole 13A of the second insulating member 13. Accordingly, conduction between the operation wire 8 and the electrode portion 9 is more secured, the operation wire 8 does not drop off from the forceps members 5 and 6, and it is possible to perform stable procedures.

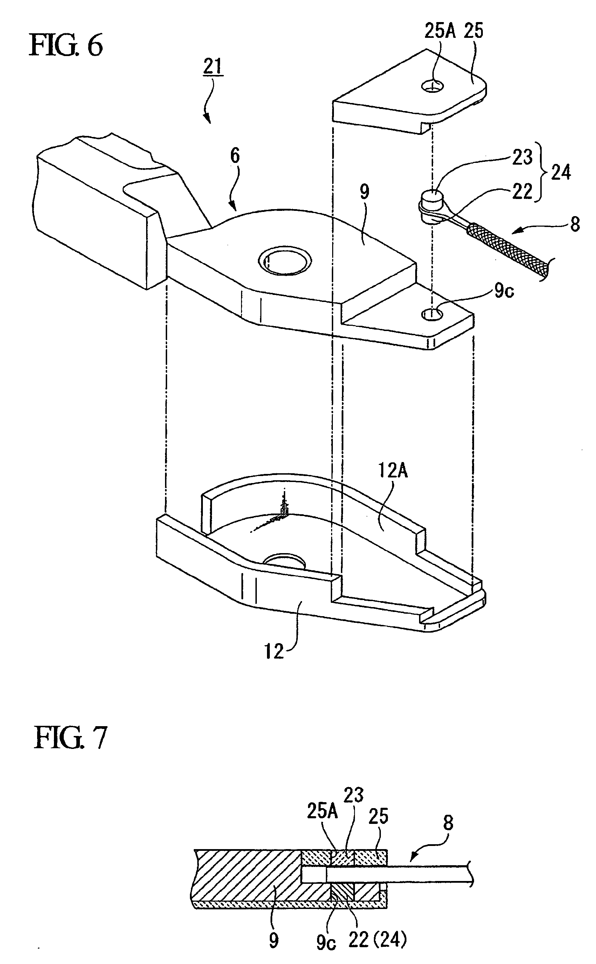

Next, a second embodiment in accordance with the present invention shall be described with reference to FIGS. 6 to 9. The difference between a high-frequency treatment instrument 21 in accordance with the present embodiment and the above-described high-frequency treatment instrument 1 is in the connection between the operation wire and the electrode portion.

Common structures with the high-frequency treatment instrument 1 in accordance with the first embodiment shall be described with the same reference numbers and detailed descriptions thereof shall be omitted.

FIG 6 is an exploded perspective view showing a connection portion connecting between a second forceps member 6 and the operation wires 8 of a high-frequency treatment instrument 21. A substantially disk shape conductive member 22 and an insulating member 23 having substantially the same cross section as the conductive member 22 are concentrically fixed to the distal end of the operation wire 8 so as to sandwich the operation wire 8. In accordance with the conductive member 22 and the insulating member 23 fixed in this way, a fitting axis 24, which rotatably connects the operation wire 8 to the electrode portion 9, is formed.

A fitting hole 9C, to which the conductive member 22 can be fit in the axial line direction, is formed in the proximal end portion of the electrode portion 9. A fitting hole 25A, to which the insulating member 23 can be fit in the axial line direction, is formed in the second insulating member 25 that insulatingly covers the electrode portion 9 so as to sandwich the electrode portion 9.

As shown in cross-section in FIG. 7, the operation wire 8 is rotatably connected to the second forceps member 6 with the conductive member 22 of the fitting axis 24 being fitted with the fitting hole 9C of the electrode portion 9 and the insulating member 23 being fitted with the fitting hole 25A of the second insulating member 25.

The high-frequency electric current supplied from the plug 20 is supplied to the electrode portion 9 by passing the operation wire 8 and the conductive member 22.

In accordance with the high-frequency treatment instrument 21 of the present embodiment, the same effects as the above-described high-frequency treatment instrument 1 can be obtained.

In accordance with the present embodiment, since the fitting axis 24 can easily be formed by using the conductive member 22 and the insulating member 23 and only the fitting hole 9C is required to be formed in the electrode portion 9. Accordingly, it is possible to improve components workability and assembability compared with the case in which the annular distal end 8A and the projection 9B are made as in the high-frequency treatment instrument 1.

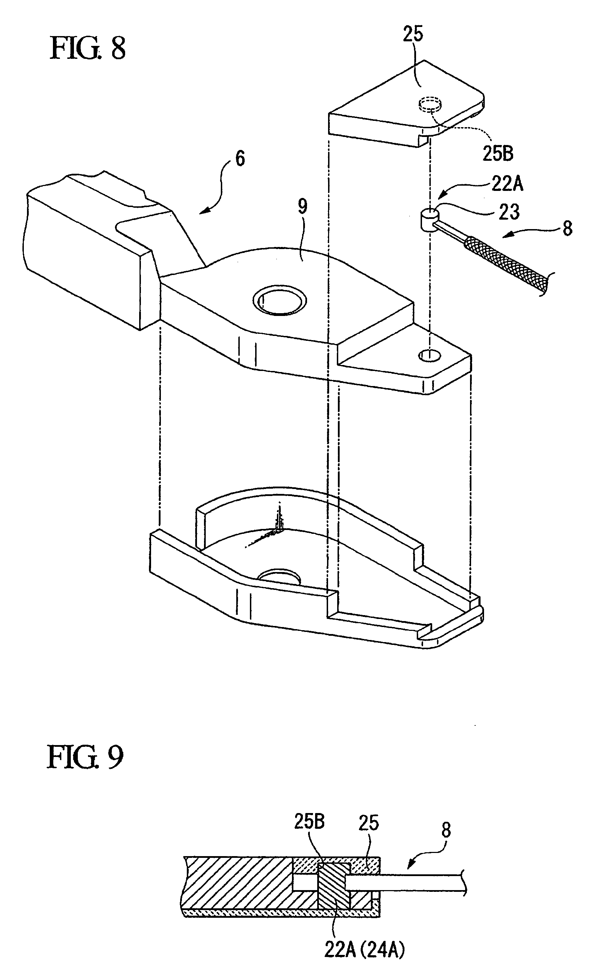

In the present embodiment, the case in which the fitting axis is formed from the conductive member and the insulating member is described. Instead of this, as shown in alternative examples in FIGS. 8 and 9, the fitting axis 24A may be formed by fixing a single conductive member 22A to the distal end of the operation wire 8. In this case, it is not necessary to concentrically coordinate the conductive member and the insulating member, it is possible to further improve components workability.

In this case, in order to assure insulation in the proximal end portion of the second forceps member 6, as shown in FIGS. 8 and 9, the fitting hole 25B of the second insulating member 25 may be formed to have a bottom without penetrating the outer surface of the second insulating member 25 so that the end surface of the fitting axis 24A is not exposed. As in the fitting hole 25A, in which the penetrating fitting hole and the fitting axis 24A are fitted, the end surface of the fitting axis 24A on the side of the fitting hole 25A may have an insulating coating or the like to assure insulation.

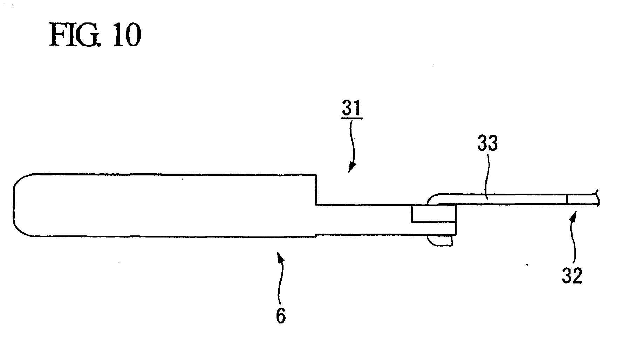

Next, a third embodiment in accordance with the present invention shall be described with reference to FIGS. 10 to 14. The difference between a high-frequency treatment instrument 31 in accordance with the present embodiment and the above-described high-frequency treatment instrument 1 is in the connection between the operation wire and the electrode portion.

Common structures with the high-frequency treatment instrument 1 in accordance with the first embodiment shall be described with the same reference numbers and detailed descriptions thereof shall be omitted.

FIG 10 shows a second forceps member 6 of the high-frequency treatment instrument 31 and an operation wire 32. As shown in FIG. 10, a predetermined length from a distal end of the operation wire 32 including the distal end surface thereof, for example a range of 20 mm or higher, is insulatingly covered with an insulating member 33. This insulating covering may be made by insulating coating or the like.

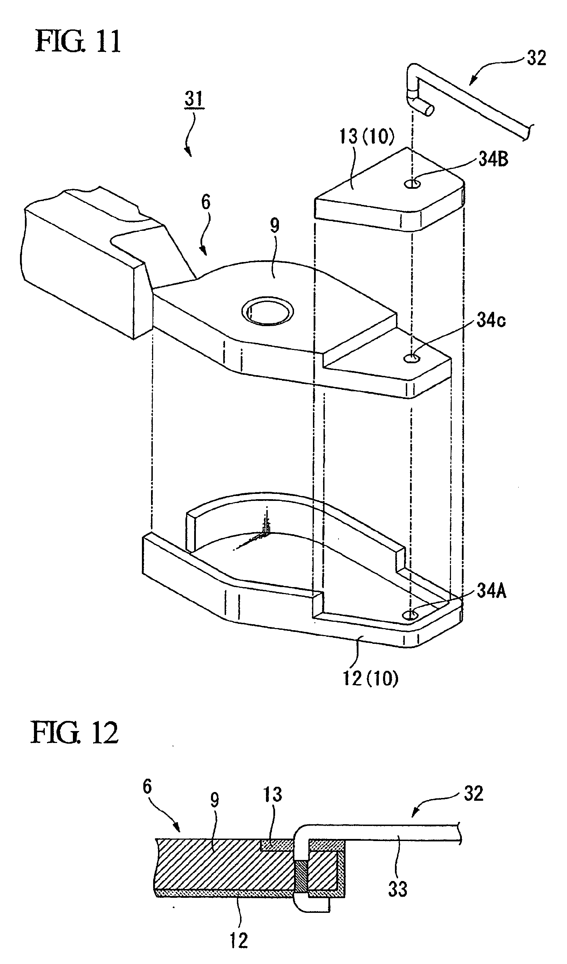

FIG 11 is a perspective exploded view showing a connection portion connecting the second forceps member 6 and the operation wire 32. As shown in FIG 11, the first insulating member 12 and the second insulating member 13 which form an insulating portion 10 and the electrode portion 9 which is sandwiched by the insulating members have penetrating holes 34A, 34B, and 34C. These 3 members are overlapped and bonded so that each of the penetrating holes 34A, 34B, and 34C are substantially coaxial.

As shown in cross-section in FIG 12, the operation wire 32 is inserted into the second forceps member 6 by penetrating each of the insulating members 12 and 13 and the electrode portion 9 so that the operation wire 32 is substantially in parallel with the rotation axis 7. The second forceps member 6 and the operation wire 32 are rotatably connected by the distal end of the operation wire 32 being projected from the first insulating member 12 and folded back.

The insulating member 33 of the operation wire 32 does not cover the connection portion with the electrode portion 9 so that a conductive surface is exposed. Accordingly, the operation wire 32 and the electrode portion 9 are electrically connected.

The diameter of the location not covered by the insulating member 33 is smaller than a location that is covered by the insulating member 33. Accordingly, the conductivity of the location may be improved by making the diameter thereof substantially the same as the location covered by the insulatingly member 33 by twisting a conductive member or the like around the location, for example.

In accordance with the high-frequency treatment instrument 31 of the present embodiment, the same effect as the above-described high-frequency treatment instrument 1 can be obtained. Since it is no longer necessary to perform complicated processes relative to the operation wire 32, it is possible to further improve assemblability.

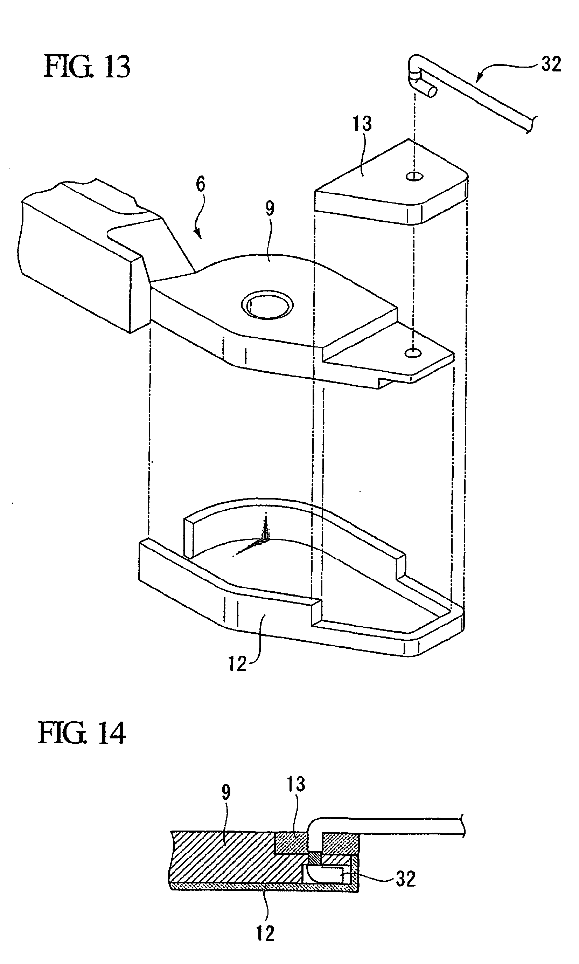

In the present embodiment, a case, in which the operation wire 32 penetrates the second forceps member 6 as a whole, is described. Instead of this, as shown in alternative cases shown in FIGS. 13 and 14, the connection portion may be made such that a part of the electrode portion 9 is made thin so that the distal end of the operation wire 32 which is folded back can be housed between the first forceps member 12 and the electrode member 9. In this manner, since the distal end of the operation wire 32 does not project, the distal end of the operation wire 32 does not touch tissues inside of the body cavity. Accordingly, it is possible to perform procedures more safely.

In the above descriptions, embodiments of the present invention have been explained, but the specific configuration is not limited to those of the above embodiments. Design modifications and the like which do not deviate from the scope of the invention are also included.

For example, in each of the above-described embodiments, a monopolar type high-frequency treatment instrument, in which, among a pair of forceps members, the treatment electrode is provided only in the second forceps member, is described.
Instead of this, the high-frequency treatment instrument of the present invention may be constituted as a bipolar type high-frequency treatment instrument, in which the electrode portions are provided in both forceps members of a pair of forceps members. In this case, another first forceps member may be made in the same manner as the second forceps member.

In each of the above-described embodiments, the insulating portion, which is constituted by using the insulating member, is described as example. Instead of this, the insulating portion may be constituted by performing the insulating coating on the outer surface of the proximal end portion of the electrode portion.

## Claims

1. A high-frequency treatment instrument (1) that is used with high-frequency electric current supplied from a power source comprising:
a pair of forceps members (5, 6) connected with each other by a rotational axis (7); and
an operation wire (8, 32) having a distal end portion which is rotationally connected to proximal end portions of the forceps members (5, 6) and a proximal end portion which is electrically connected with the power source, wherein
at least one of the pair of forceps members (5, 6) has
a conductive electrode portion (9), and
an insulating portion (10) disposed so as to cover at least part of the electrode portion (9),
the operation wire (8, 32) is electrically connected with the electrode portions (9), and
**characterized in that** the insulating portion (10) has a first insulating member (12), the figure of which corresponds to the electrode portion (9), and a second insulating member (13, 25), and
a conductive outer surface of the forceps members (5, 6), which includes a portion located closer to the proximal end portions of the forceps members (5, 6) than the rotational axis of the forceps members (5, 6) where the operation wire (8, 32) and the electrode portion (9) are electrically connected such a portion being sandwiched and covered by the first insulating member (12) and second insulating member (13, 25) so as not to be exposed.

2. The high-frequency treatment instrument (1) according to Claim 1, wherein the operation wire (8, 32) has an annular connection portion (8A) which is formed in the distal end thereof,
the electrode portion (9) has a locking projection (9B) formed closer to the proximal end portion than the rotational axis,
the operation wire (8, 32) and the electrode portion (9) are electrically connected by the locking projection (9B) being inserted through the connection portion (8A).

3. The high-frequency treatment instrument (1) according to Claim 1, wherein the electrode portion (9) has a fitting hole (13A) formed closer to the proximal end portion than the rotational axis,
the operation wire (8, 32) has a fitting axis (24) attached to the distal end portion thereof, and
the operation wire (8, 32) and the electrode portion (9) are electrically connected by the fitting axis (24) which is rotationally fit into the fitting hole (13A).

4. A high-frequency treatment instrument (1) according to claim 1 wherein a penetrating hole (34A, 34B, 34C) penetrating the insulating portion (10) and the electrode portion (9) is formed in the proximal end portion of the forceps member (5, 6) having the electrode portion(9), and
an area including at least the distal end of the operation wire (8, 32) except a portion located in the penetrating hole (34A, 34B, 34C) is insulatingly covered and inserted through the penetrating hole (34A, 34B, 34C) and the electrode portion are electrically connected in the penetrating hole (34A, 34B, 34C).

## Patentansprüche

1. Hochfrequenzbehandlungsinstrument (1), das mit einem hochfrequentigen elektrischen Strom verwendet wird, der von einer Stromquelle zugeführt wird, aufweisend:
ein Paar von Zangenelementen (5, 6), die miteinander über eine Rotationsachse (7) verbunden sind; und
einen Betätigungsdraht (8, 32) mit einem distalen Endabschnitt, der drehbar mit dem proximalen Endabschnitten der Zangenelemente (5, 6) verbunden ist, und einem proximalen Endabschnitt, der elektrisch mit der Stromquelle verbunden ist, wobei wenigstens eines der Zangenelemente des Paars von Zangenelementen (5, 6) einen leitenden Elektrodenabschnitt (9) und einen Isolierungsabschnitt (10) aufweist, der zum Abdecken wenigstens eines Abschnitts des Elektrodenabschnitts (9) angeordnet ist,
der Betätigungsdraht (8, 32) elektrisch mit den Elektrodenabschnitten (9) verbunden ist, und
**dadurch gekennzeichnet, dass** der Isolierungsabschnitt (10) ein erstes Isolierungselement (12), dessen Form der Form des Elektrodenabschnitts (9) entspricht, und ein zweites Isolierungselemente (13, 25) umfasst, und
eine leitende Außenfläche der Zangenelemente (5, 6), die einen näher an den proximalen Endabschnitten der Zangenelemente (5, 6) als an der Rotationsachse der Zangenelemente (5, 6) angeordneten Abschnitt umfasst, wo der Betätigungsdraht (8, 32) und der Elektrodenabschnitt (9) elektrisch verbunden sind, wobei ein derartiger Abschnitt zwischen dem ersten Isolierungselement (12) und dem zweiten Isolierungselement (13) geklemmt und abgedeckt ist, so dass er nicht frei liegt.

2. Hochfrequenzbehandlungsinstrument (1) nach Anspruch 1, wobei der Betätigungsdraht (8, 32) einen ringförmigen Verbindungsabschnitt (8A) aufweist, der an einem distalen Ende ausgebildet ist,
der Elektrodenabschnitt (9) einen Verriegelungsvorsprung (9B) aufweist, der näher an dem proximalen Endabschnitt als an der Rotationsachse ausgebildet ist,
der Betätigungsdraht (8, 32) und der Elektrodenabschnitt (9) elektrisch mittels dem Verriegelungsvorsprung (9B) verbunden sind, der durch den Verbindungsabschnitt (8A) geführt ist.

3. Hochfrequenzbehandlungsinstrument (1) nach Anspruch 1, wobei der Elektrodenabschnitt (9) eine Passöffnung (13A) aufweist, die näher an dem proximalen Ende als an der Rotationsachse ausgebildet ist,
der Betätigungsdraht (8, 32) eine Passachse (24) aufweist, die an dem distalen Endabschnitt angeordnet ist, und
der Betätigungsdraht (8, 32) und der Elektrodenabschnitt (9) mittels der Passachse (24) elektrisch verbunden sind, die rotatorisch in die Passöffnung eingepasst ist.

4. Hochfrequenzbehandlungsinstrument (1) nach Anspruch 1, wobei eine Durchdringungsöffnung (34A, 34B, 34C), die den Isolierungsabschnitt (10) und den Elektrodenabschnitt (9) durchdringt, an dem proximalen Endabschnitt der Zangenelemente (5, 6) ausgebildet ist, die den Elektrodenabschnitt (9) aufweisen, und
ein wenigstens das distale Ende des Betätigungsabschnitt (8, 32) bis auf einen in der Durchdringungsöffnung (34A, 34B, 34C) angeordneten Abschnitt umfassender Bereich isolierend abgedeckt und in die Durchdringungsöffnung (34A, 34B, 34C) eingeführt ist, und der Elektrodenabschnitt elektrisch mit der Durchdringungsöffnung (34A, 34B, 34C) verbunden ist.

## Revendications

1. Instrument de traitement haute fréquence (1) qui est utilisé avec un courant électrique haute fréquence délivré depuis une source d'alimentation comprenant :
une paire d'éléments de pince (5, 6) raccordés l'un à l'autre par un axe de rotation (7) ; et
un câble d'actionnement (8, 32) comportant une partie d'extrémité distale qui est raccordée en rotation aux parties d'extrémité proximales des éléments de pince (5, 6) et une partie d'extrémité proximale qui est électriquement connectée à la source d'alimentation, dans lequel
au moins l'un parmi la paire d'éléments de pince (5, 6) comporte
une partie d'électrode conductrice (9), et
une partie isolante (10) disposée de façon à couvrir au moins une partie de la partie d'électrode (9),
le câble d'actionnement (8, 32) est électriquement connecté avec la partie d'électrode (9), et
**caractérisé en ce que** la partie isolante (10) comporte un premier élément isolant (12), dont la forme correspond à la partie d'électrode (9), et un deuxième élément isolant (13, 25), et
une surface externe conductrice des éléments de pince (5, 6), qui comprend une partie placée plus près des parties d'extrémité proximales des éléments de pince (5, 6) que de l'axe de rotation des éléments de pince (5, 6) où le câble d'actionnement (8, 32) et la partie d'électrode (9) sont électriquement connectés comme une partie étant prise en sandwich et recouverte par le premier élément isolant (12) et le deuxième élément isolant (13, 25) de façon à ne pas être exposée.

2. Instrument de traitement haute fréquence (1) selon la revendication 1, dans lequel le câble d'actionnement (8, 32) comporte une partie de raccordement annulaire (8A) qui est formée dans l'extrémité distale de celui-ci,
la partie d'électrode (9) comporte une saillie de blocage (9B) formée plus près de la partie d'extrémité proximale que de l'axe de rotation,
le câble d'actionnement (8, 32) et la partie d'électrode (9) sont électriquement connectés par la saillie de blocage (9B) étant insérée à travers la partie de raccordement (8A).

3. Instrument de traitement haute fréquence (1) selon la revendication 1, dans lequel la partie d'électrode (9) comporte un trou d'assemblage (13A) formé plus près de la partie d'extrémité proximale que de l'axe de rotation,
le câble d'actionnement (8, 32) comporte un axe d'assemblage (24) fixé à la partie d'extrémité distale de celui-ci, et
le câble d'actionnement (8, 32) et la partie d'électrode (9) sont électriquement connectés par l'axe d'assemblage (24) qui est ajusté en rotation dans le trou d'assemblage (13A).

4. Instrument de traitement haute fréquence (1) selon la revendication 1, dans lequel un trou de pénétration (34A, 34B, 34C) pénétrant la partie isolante (10) et la partie d'électrode (9) est formé dans la partie d'extrémité proximale des éléments de pince (5, 6) comportant la partie d'électrode (9), et
une zone incluant au moins l'extrémité distale du câble d'actionnement (8, 32) exceptée une partie placée dans le trou de pénétration (34A, 34B, 34C) est couverte de manière isolante et insérée à travers le trou de pénétration (34A, 34B, 34C) et la partie d'électrode sont électriquement connectées dans le trou de pénétration (34A, 34B, 34C).
